(19) **Europäisches Patentamt / European Patent Office / Office européen des brevets**

(11) **EP 4 644 887 A1**

(12) **EUROPEAN PATENT APPLICATION**

(43) Date of publication:
**05.11.2025  Bulletin 2025/45**

(21) Application number: **25167311.7**

(22) Date of filing: **31.03.2025**

(51) International Patent Classification (IPC):
**G01N 23/046** (2018.01)    **A61B 6/42** (2024.01)
**G01T 1/29** (2006.01)    **G06T 11/00** (2006.01)

(52) Cooperative Patent Classification (CPC):
**G01N 23/046; A61B 6/4241; G01T 1/2985; G06T 11/005**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **25.04.2024  US 202418646566**

(71) Applicant: **GE Precision Healthcare LLC Waukesha, WI 53188 (US)**

(72) Inventors:
- **KIM, Changlyong**
  **Waukesha, 53188-1696 (US)**
- **ZHANG, Fengchao**
  **Waukesha, 53188-1696 (US)**
- **CEDERSTRÖM, Björn**
  **10691 Stockholm (SE)**
- **CURTIS, Tyler**
  **Waukesha, 53188-1696 (US)**
- **BOUDRY, John**
  **Waukesha, 53188-1696 (US)**

(74) Representative: **AWA Sweden AB Box 45086 104 30 Stockholm (SE)**

(54) **METHOD FOR REDUCING DEPENDENCE ON FOCAL SPOT SIZE IN MATERIAL DENSITY CALIBRATION**

(57)    Methods and systems are provided for calibrating a photon counting computed tomography (PCCT) system (100, 200). To reduce an amount of calibration data stored in a memory of the PCCT system (100, 200), and to reduce a time spent calibrating the PCCT system (100, 200), a method (600, 700) is provided for using a material decomposition (MD) calibration vector generated for a first focal spot size to correct projection data acquired using the PCCT system at different focal spot sizes. To compensate for spectral differences due to focal spot size, the projection data is corrected and normalized by air calibration vectors generated for each different focal spot size.

FIG. 7

**Description**

TECHNICAL FIELD

Embodiments of the subject matter disclosed herein relate to imaging systems and methods, and more particularly, to calibration of computerized tomography (CT) imaging systems.

BACKGROUND

In computed tomography (CT) imaging systems, an electron beam generated by a cathode is directed towards a target within an X-ray tube. A fan-shaped or cone-shaped beam of X-rays produced by electrons colliding with the target is directed towards a subject, such as a patient. After being attenuated by the object, the X-rays impinge upon an array of X-ray detectors, generating a CT image. A quality of a CT image may be increased by using Photon Counting CT (PCCT), where the X-ray detectors are photon counting detectors, and photons are counted to provide spectral information.

To ensure that an image reconstructed from the PCCT scan will be accurate, the CT system may be calibrated periodically and/or regularly. During calibration, various calibration vectors may be generated, which are used to compensate for a variation in detector response, which may depend on a scan protocol, a focal spot size, a bowtie filter used, or a selected X-ray peak energy. The calibration vectors may be applied to the detector responses to adjust photon counts to increase image quality. However, generating the various calibration vectors may be a time-consuming process, during which the CT system may not be available for use on subjects.

SUMMARY

The current disclosure at least partially addresses one or more of the above identified issues by a method for a photon counting computed tomography (PCCT) system, the method comprising performing a scan using the PCCT system, with a first focal spot of a first size; applying a material decomposition (MD) calibration vector to correct projection data acquired during the scan, the material decomposition (MD) calibration vector generated for a second focal spot of a second size, the second size different from the first size; reconstructing an image from the corrected projection data; and displaying the image on a display device.

The above advantages and other advantages, and features of the present description will be readily apparent from the following Detailed Description when taken alone or in connection with the accompanying drawings. It should be understood that the summary above is provided to introduce in simplified form a selection of concepts that are further described in the detailed description. It is not meant to identify key or essential features of the claimed subject matter, the scope of which is defined uniquely by the claims that follow the detailed description. Furthermore, the claimed subject matter is not limited to implementations that solve any disadvantages noted above or in any part of this disclosure.

BRIEF DESCRIPTION OF THE DRAWINGS

Various aspects of this disclosure may be better understood upon reading the following detailed description and upon reference to the drawings in which:

FIG. 1 shows a pictorial view of a photon counting computed tomography (PCCT) imaging system, in accordance with one or more embodiments of the present disclosure;
FIG. 2 shows a block schematic diagram of an example PCCT imaging system, in accordance with one or more embodiments of the present disclosure;
FIG. 3A is a schematic diagram of an exemplary X-ray tube, in accordance with one or more embodiments of the present disclosure;
FIG. 3B is a schematic diagram of a focal spot on a target, in accordance with one or more embodiments of the present disclosure;
FIG. 4 shows a distribution of electrons of an electron beam of a PCCT system focused on four discrete focal spots, in accordance with one or more embodiments of the present disclosure;
FIG. 5 is a flowchart illustrating a method for calibrating a PCCT system, in accordance with one or more embodiments of the present disclosure;
FIG. 6 is a flowchart illustrating a method for correcting projection data acquired via a PCCT system using calibration vectors generated during calibration of the PCCT system, in accordance with one or more embodiments of the present disclosure;
FIG. 7 is a flowchart illustrating a method for applying calibration vectors to projection data acquired via a PCCT system, in accordance with one or more embodiments of the present disclosure;

FIG. 8 is a first exemplary image of a phantom after a first correction of projection data using calibration vectors, in accordance with one or more embodiments of the present disclosure;

FIG. 9 is a second exemplary image of a phantom after a second correction of projection data using calibration vectors, in accordance with one or more embodiments of the present disclosure; and

FIG. 10 is a third exemplary image of a phantom after a third correction of projection data using calibration vectors, in accordance with one or more embodiments of the present disclosure.

The drawings illustrate specific aspects of the described systems and methods. Together with the following description, the drawings demonstrate and explain the structures, methods, and principles described herein. In the drawings, the size of components may be exaggerated or otherwise modified for clarity. Well-known structures, materials, or operations are not shown or described in detail to avoid obscuring aspects of the described components, systems and methods.

DETAILED DESCRIPTION

This description and embodiments of the subject matter disclosed herein relate to methods and systems for reducing an amount of time spent calibrating a photon counting computed tomography (PCCT) system. Typically, in computed tomography (CT) imaging systems, an X-ray source or X-ray tube emits a fan-shaped beam or a cone-shaped beam towards an object, such as a patient. Generally, in CT systems the X-ray source and the detector array are rotated about a gantry within an imaging plane and around the patient, and images are generated from projection data at a plurality of views at different view angles. For example, for one rotation of the X-ray source, 1000 views may be generated by the CT system. The beam, after being attenuated by the patient, impinges upon an array of radiation detectors. The X-ray detector or detector array typically includes a collimator for collimating X-ray beams received at the detector, a scintillator disposed adjacent to the collimator for converting X-rays to light energy, and photodiodes for receiving the light energy from the adjacent scintillator and producing electrical signals therefrom. An intensity of the attenuated X-ray beam radiation received at the detector array is typically dependent upon the attenuation of the X-ray beam by the patient. Each detector element of a detector array produces a separate electrical signal indicative of the attenuated beam received by each detector element. The electrical signals are transmitted to a data processing system for analysis. The data processing system processes the electrical signals to facilitate generation of an image.

Conventional CT imaging systems utilize detectors that convert radiographic energy into current signals that are integrated over a time period, then measured and ultimately digitized. However, a drawback of such detectors is their inability to provide data or feedback as to the number and/or energy of photons detected. In contrast, PCCT detectors may provide photon counting and/or energy discriminating feedback with high spatial resolution. PCCT detectors can be caused to operate in an X-ray counting mode, an energy measurement mode of each X-ray event, or both.

However, it may be difficult to measure PCCT detector response accurately, for different reasons. One reason is photon count "pile-up", a phenomenon that occurs when a source flux at a detector is so high that there is a non-negligible possibility that two or more X-ray photons deposit their energy in a single pixel close enough in time so that their signals overlap with each other. Pile-up phenomenon are of two general types, which result in somewhat different effects. In the first type, the two or more events are separated by sufficient time so that they are recognized as distinct events, but the signals overlap so that the precision of the measurement of the energy of the later arriving X-ray or X-rays is degraded. This type of pile-up results in a degradation of the energy resolution of the system. In the second type of pile-up, the two or more events arrive close enough in time so that the system is not able to resolve them as distinct events. In such a case, these events are recognized as one single event having the sum of their energies and the events are shifted in the spectrum to higher energies. In addition, pile-up leads to a more or less pronounced depression of counts in high X-ray flux, resulting in detector quantum efficiency (DQE) loss.

Therefore, an output of the photon counting detectors is typically corrected for pile-up effects. The pile-up may occur at different detectors, depending on a size and density of an anatomical region being scanned. To correct for the pile-up effects, measured photon counts at each detector may be adjusted by applying a pile-up calibration vector. The pile-up calibration vector includes values that indicate, for each individual detector element (e.g., pixel), how much the response of the detector should be adjusted to account for the pile-up.

PCCT detector responses may also vary and need to be corrected for other reasons. For example, an amount of tube current supplied by the X-ray tube during a scan may not precisely match an amount of tube current requested for the scan. To correct for variations in the tube current, a tube current calibration vector may be applied to the detector response. The tube current calibration vector functions as a lookup table that indicates, for each detector, how the tube current should be adjusted based on an amount of requested tube current.

Additionally, the span of each detector element in a detector array may vary. To obtain accurate projection data, signals from a plurality of detector elements may be normalized pixel-by-pixel by applying a third, air calibration vector. A fourth, material decomposition (MD) calibration vector may be applied to adjust the photon counts based on a ratio of component materials in a scanned anatomy. Other types of calibration vectors may also be applied.

The pile-up, tube current, air, MD, and other calibration vectors are typically generated or updated for a given set of protocols during a calibration stage of the PCCT system prior to performing a scan. During the calibration stage, various scans may be performed on one or more phantoms, in accordance with various protocols. Each protocol may specify a set of desired parameters for the scan, such as focal spot size, tube current, collimation parameters, and the like. After each scan or a set of scans, a calibration vector may be created based on detector outputs during the scan.

Thus, achieving a desired image quality from the PCCT system typically entails generating various sets of calibration vectors which can be selectively applied to PCCT system detector outputs to compensate and correct for pixel-by-pixel variance in detector responses. The sets of calibration vectors may be generated during periodic (e.g., yearly) calibrations performed on the PCCT system. The generation of calibration vectors during yearly calibrations may be performed in a detailed fashion, which may entail performing a large number of acquisition scans on lots of different phantoms, using various combinations of scan parameters. When a scan is subsequently performed on a subject, appropriate calibration vectors are selected and applied based on a protocol used for the scan and physical properties of a scanned anatomy. The more phantoms and combinations used, the more calibration vectors may be created, which may result in an overall increase in a quality of an image reconstructed using the PCCT system.

However, as a number of scans increases, more time is consumed by the calibration. Thus, there is a tradeoff between generating a desired number of scans/calibration vectors to meet an image quality goal, and maintaining a total calibration time within a desired range. Currently, a yearly calibration may take multiple days, which results in a customer downtime that is greater than desired.

The calibration vectors may additionally be regenerated or updated during regular calibrations performed on the PCCT system. During the regular calibrations, scans may be performed using a smaller number of combinations of scanning parameters than in the yearly calibrations, to reduce the calibration time. For example, the regular calibrations may be performed daily, where a daily calibration may take 30-60 minutes.

During both the periodic and regular calibrations, resources of the PCCT system may not be available for performing scans on subjects. As a result, to increase a use and an availability of the PCCT system, it is desirable to reduce the amount of time spent performing the calibrations, while still generating a sufficient number of calibration vectors to achieve a desired image quality for the expected patient scan protocols.

While some types of calibrations, such as pileup calibrations, are independent of one or more protocol settings, other types of calibration need to be repeated for each protocol. For example, the detector response of air, tube current and MD calibration depends on focal spot sizes due to, mostly, the X-ray beam width, its intensity variation, and detector structure. The focal spot sizes may range from extra-small (XS), to small (S), to large (L), to extra-large (XL). The repetition of those focal spot size dependent calibrations can be very costly for some calibration in both calibration data time and processing time. For example, air and tube current calibration needs only one scan for each focal spot size, but for MD, a set of multiple scans are necessary for each focal spot size.

In particular, generating the MD calibration vectors may be more complex and time-consuming than other types of calibration vectors, because the MD calibration is based on scans of varying combinations of two or more reference slab materials, such as polyethylene (PE) and aluminum (Al) or PE and polyvinylchloride (PVC). PE represents soft tissue in human body and Al is used to represent bone. For example, five different thicknesses of PE and four different thicknesses of Al may be used to cover human X-ray attenuation. Combining these different thicknesses may entail up to 20 (e.g., 5x4) calibration scans for one focal spot size MD vector. For four focal spot sizes, it can be up to 20x4=100 scans. The number of scans can quickly increase depending on the number of slabs and their combination. In addition, for thicker combinations of reference slabs causing high X-ray attenuation, the calibration scan time during which the X-ray is on may increase to collect enough X-ray counts, which may increase the data size and its handling time, and may reduce a lifetime of the X-ray tube. Thus, a large proportion of a total amount of time spent calibrating the PCCT system may be spent on generating the MD calibration vectors, with a smaller proportion of the total amount of time being spent on generating other types of calibration vectors.

To reduce the total amount of time spent calibrating the PCCT system, systems and methods are disclosed herein to decrease the amount of time spent generating the MD calibration vectors during calibrations. Specifically, the disclosed approach reduces a total number of MD calibration scans and the corresponding vectors that are relied on by the PCCT system for material density decomposition in image reconstruction. Rather than relying on different sets of calibration vectors for each focal spot size, one or more common sets of MD calibration vectors may be generated that may be applied to various focal spot sizes during image reconstruction. By using the common sets of MD calibration vectors for the projection data acquired at each focal spot size, a time spent performing the calibration vector generation and an amount of MD calibration data collection may be decreased by as much as 60%, while maintaining image quality within a desired range. By reducing the time spent performing the calibrations, the availability of the PCCT system for use on patient scans may be increased. Additionally, an amount of computational and memory resources consumed by the PCCT system may be reduced, thereby increasing an availability of the resources for other tasks, and increasing an efficiency of the use of the PCCT system.

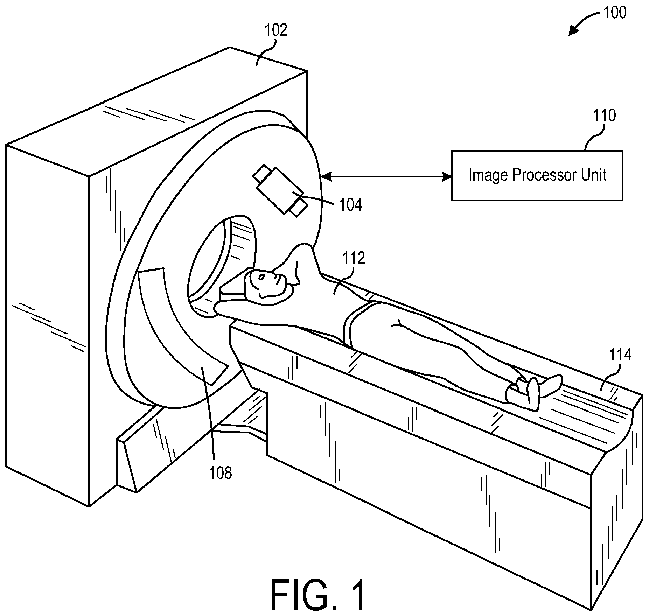

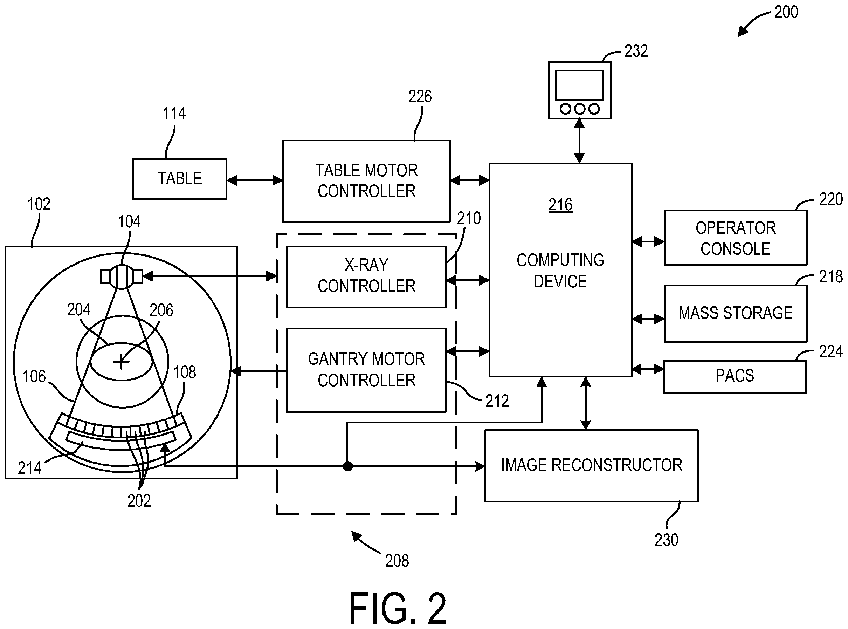

FIG. 1 illustrates an exemplary PCCT system 100 configured for CT imaging with photon counting detectors. Particularly, the PCCT system 100 is configured to image a subject 112 such as a patient, an inanimate object, one or more manufactured parts, and/or foreign objects such as dental implants, stents, and/or contrast agents present within the body. In one embodiment, the PCCT system 100 includes a gantry 102, which in turn, may further include at least one X-ray source 104 configured to project a beam of X-ray radiation 106 (see FIG. 2) for use in imaging the subject 112 laying on a table 114. Specifically, the X-ray source 104 is configured to project the X-ray radiation beams 106 towards a detector array 108 positioned on the opposite side of the gantry 102. Although FIG. 1 depicts a single X-ray source 104, in certain embodiments, multiple X-ray sources and detectors may be employed to project a plurality of X-ray radiation beams for acquiring projection data at different energy levels corresponding to the patient. In some embodiments, the X-ray source 104 may enable dual-energy gemstone spectral imaging (GSI) by rapid peak kilovoltage (kVp) switching. In the embodiments described herein, the X-ray detector employed is a photon counting detector which is capable of differentiating X-ray photons of different energies.

In certain embodiments, the PCCT system 100 further includes an image processor unit 110 configured to reconstruct images of a target volume of the subject 112 using an iterative or analytic image reconstruction method. For example, the image processor unit 110 may use an analytic image reconstruction approach such as filtered back projection (FBP) to reconstruct images of a target volume of the patient. As another example, the image processor unit 110 may use an iterative image reconstruction approach such as advanced statistical iterative reconstruction (ASIR), conjugate gradient (CG), maximum likelihood expectation maximization (MLEM), model-based iterative reconstruction (MBIR), and so on to reconstruct images of a target volume of the subject 112. As described further herein, in some examples the image processor unit 110 may use both an analytic image reconstruction approach such as FBP in addition to an iterative image reconstruction approach.

In some CT imaging system configurations, an X-ray source projects a cone-shaped X-ray radiation beam which is collimated to lie within an X-Y-Z plane of a Cartesian coordinate system and generally referred to as an "imaging plane." The X-ray radiation beam passes through an object being imaged, such as the patient or subject. The X-ray radiation beam, after being attenuated by the object, impinges upon an array of detector elements. The intensity of the attenuated X-ray radiation beam received at the detector array is dependent upon the attenuation of an X-ray radiation beam by the object. Each detector element of the array produces a separate electrical signal that is a measurement of the X-ray beam attenuation at the detector location. The attenuation measurements from all the detector elements are acquired separately to produce a transmission profile.

In some CT systems, the X-ray source and the detector array are rotated with a gantry within the imaging plane and around the object to be imaged such that an angle at which the X-ray beam intersects the object constantly changes. A group of X-ray radiation attenuation measurements, e.g., projection data, from the detector array at one gantry angle is referred to as a "view." A "scan" of the object includes a set of views made at different gantry angles, or view angles, during one revolution of the X-ray source and detector.

FIG. 2 illustrates an exemplary imaging system 200 similar to the PCCT system 100 of FIG. 1. In accordance with aspects of the present disclosure, the imaging system 200 is configured for imaging a subject 204 (e.g., the subject 112 of FIG. 1). In one embodiment, the imaging system 200 includes the detector array 108 (see FIG. 1). The detector array 108 further includes a plurality of detector elements 202 that together sense the X-ray radiation beam 106 (see FIG. 2) that pass through the subject 204 (such as a patient) to acquire corresponding projection data. In some embodiments, the detector array 108 may be fabricated in a multislice configuration including the plurality of rows of cells or detector elements 202, where one or more additional rows of the detector elements 202 are arranged in a parallel configuration for acquiring the projection data.

In certain embodiments, the imaging system 200 is configured to traverse different angular positions around the subject 204 for acquiring desired projection data. Accordingly, the gantry 102 and the components mounted thereon may be configured to rotate about a center of rotation 206 for acquiring the projection data, for example, at different energy levels. Alternatively, in embodiments where a projection angle relative to the subject 204 varies as a function of time, the mounted components may be configured to move along a general curve rather than along a segment of a circle.

As the X-ray source 104 and the detector array 108 rotate, the detector array 108 collects data of the attenuated X-ray beams. The data collected by the detector array 108 undergoes pre-processing and calibration to condition the data to represent the line integrals of the attenuation coefficients of the scanned subject 204. The processed data are commonly called projections. In some examples, the individual detectors or detector elements 202 of the detector array 108 may include photon counting detectors which register the interactions of individual photons into one or more energy bins.

The acquired sets of projection data may be used for basis material decomposition (BMD). During BMD, the measured projections are converted to a set of material-density projections by applying MD calibration vectors. The material-density projections may be reconstructed to form a pair or a set of material-density maps or images of each respective basis material, such as bone, soft tissue, and/or contrast agent maps. The density maps or images may be, in turn, associated to form a 3D volumetric image of the basis material, for example, bone, soft tissue, and/or contrast agent, in the imaged volume.

Once reconstructed, the basis material image produced by the imaging system 200 reveals internal features of the subject 204, expressed in the densities of two basis materials. The density image may be displayed to show these features. In traditional approaches to diagnosis of medical conditions, such as disease states, and more generally of medical events, a radiologist or physician would consider a hard copy or display of the density image to discern characteristic features of interest. Such features might include lesions, sizes and shapes of particular anatomies or organs, and other features that would be discernable in the image based upon the skill and knowledge of the individual practitioner.

In one embodiment, the imaging system 200 includes a control mechanism 208 to control movement of the components such as rotation of the gantry 102 and the operation of the X-ray source 104. In certain embodiments, the control mechanism 208 further includes an X-ray controller 210 configured to provide power and timing signals to the X-ray source 104. Additionally, the control mechanism 208 includes a gantry motor controller 212 configured to control a rotational speed and/or position of the gantry 102 based on imaging requirements.

In certain embodiments, the control mechanism 208 further includes a data acquisition system (DAS) 214 configured to sample analog data received from the detector elements 202 and convert the analog data to digital signals for subsequent processing. The DAS 214 may be further configured to selectively aggregate analog data from a subset of the detector elements 202 into so-called macro-detectors, as described further herein. The data sampled and digitized by the DAS 214 is transmitted to a computer or computing device 216. In one example, the computing device 216 stores the data in a storage device 218. The storage device 218, for example, may be any type of non-transitory memory and may include a hard disk drive, a floppy disk drive, a compact disk-read/write (CD-R/W) drive, a Digital Versatile Disc (DVD) drive, a flash drive, and/or a solid-state storage drive.

Additionally, the computing device 216 provides commands and parameters to one or more of the DAS 214, the X-ray controller 210, and the gantry motor controller 212 for controlling system operations such as data acquisition and/or processing. In certain embodiments, the computing device 216 controls system operations based on operator input. The computing device 216 receives the operator input, for example, including commands and/or scanning parameters via an operator console 220 operatively coupled to the computing device 216. The operator console 220 may include a keyboard (not shown) or a touchscreen to allow the operator to specify the commands and/or scanning parameters.

Although FIG. 2 illustrates one operator console 220, more than one operator console may be coupled to the imaging system 200, for example, for inputting or outputting system parameters, requesting examinations, plotting data, and/or viewing images. Further, in certain embodiments, the imaging system 200 may be coupled to multiple displays, printers, workstations, and/or similar devices located either locally or remotely, for example, within an institution or hospital, or in an entirely different location via one or more configurable wired and/or wireless networks such as the Internet and/or virtual private networks, wireless telephone networks, wireless local area networks, wired local area networks, wireless wide area networks, wired wide area networks, etc.

In one embodiment, for example, the imaging system 200 either includes, or is coupled to, a picture archiving and communications system (PACS) 224. In an exemplary implementation, the PACS 224 is further coupled to a remote system such as a radiology department information system, hospital information system, and/or to an internal or external network (not shown) to allow operators at different locations to supply commands and parameters and/or gain access to the image data.

The computing device 216 uses the operator-supplied and/or system-defined commands and parameters to operate a table motor controller 226, which in turn, may control a table 114 which may be a motorized table. Specifically, the table motor controller 226 may move the table 114 for appropriately positioning the subject 204 in the gantry 102 for acquiring projection data corresponding to the target volume of the subject 204.

As previously noted, the DAS 214 samples and digitizes the projection data acquired by the detector elements 202. Subsequently, an image reconstructor 230 uses the sampled and digitized X-ray data to perform high-speed reconstruction. Although FIG. 2 illustrates the image reconstructor 230 as a separate entity, in certain embodiments, the image reconstructor 230 may form part of the computing device 216. Alternatively, the image reconstructor 230 may be absent from the imaging system 200 and instead the computing device 216 may perform one or more functions of the image reconstructor 230. Moreover, the image reconstructor 230 may be located locally or remotely, and may be operatively connected to the imaging system 200 using a wired or wireless network. Particularly, one exemplary embodiment may use computing resources in a "cloud" network cluster for the image reconstructor 230.

In one embodiment, the image reconstructor 230 stores the images reconstructed in the storage device 218. Alternatively, the image reconstructor 230 may transmit the reconstructed images to the computing device 216 for generating useful patient information for diagnosis and evaluation. In certain embodiments, the computing device 216 may transmit the reconstructed images and/or the patient information to a display or display device 232 communicatively coupled to the computing device 216 and/or the image reconstructor 230. In some embodiments, the reconstructed images may be transmitted from the computing device 216 or the image reconstructor 230 to the storage device 218 for short-term or long-term storage.

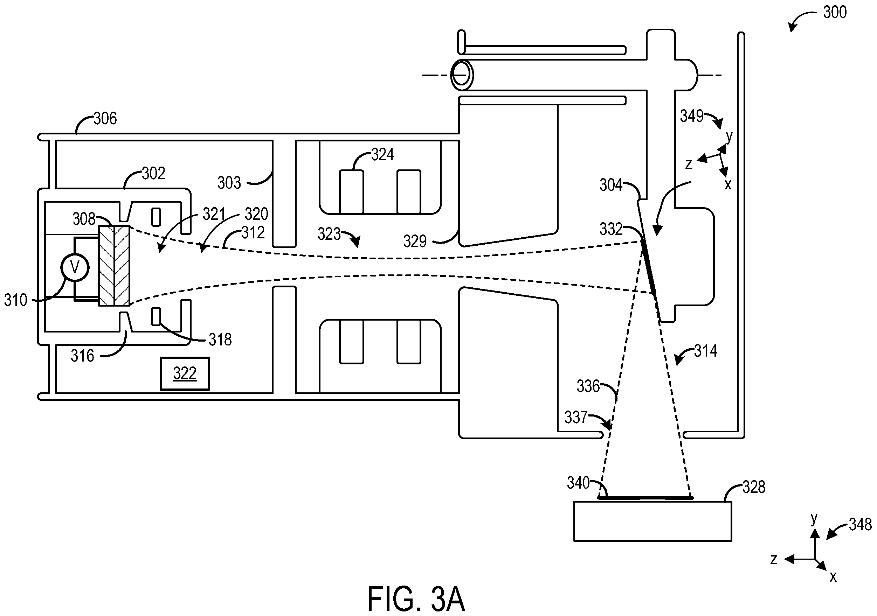

Referring now to FIG. 3A, an exemplary X-ray tube 300 is shown. In one embodiment, the X-ray tube 300 may be the X-ray source 104 (see FIGS. 1-2). In the illustrated embodiment, the X-ray tube 300 includes an exemplary cathode 302 and an anode 303 disposed within a tube casing 306. The cathode may include one or more emitters 308. In the present example, the cathode 302, and in particular the one or more emitters 308, may be directly heated by passing a current through the one or more emitters 308, which may be supplied by a voltage source 310. In one embodiment, a current of about 10 amps (A) may be passed through the one or more emitters 308. The one or more emitters 308 may emit an electron beam 312 as a result of being heated by the current supplied by the voltage source 310. As used herein, the term "electron beam" may be used to refer to a stream of electrons that have substantially similar velocities.

The electron beam 312 may be directed towards a target 304 to produce X-rays 314. More particularly, the electron beam 312 may be accelerated from the emitter 308 towards the target 304 by applying a potential difference between the one or more emitters 308 and the anode 303. In one embodiment, a high voltage in a range from about 40 kV to about 450 kV may be applied to set up the potential difference between the one or more emitters 308 and the anode 303, thereby generating one or more electric fields 320 in the X-ray tube 300.

The electron beam 312 may impinge on the target 304 at a focal spot 332. Focal spot 332 may have an orientation indicated by coordinate axes 349. When the electron beam 312 impinges upon the target 304, heat may be generated in the target 304 at a location of the focal spot 332, which may be significant enough to melt the target 304. In various embodiments, a rotating target may be used to circumvent the problem of heat generation in the target 304. For example, the target 304 may be configured to rotate such that the focal spot 332 generated by the electron beam 312 striking the target 304 does not strike the target 304 consistently at the same location, so that the target 304 may not melt. In various embodiments, the target 304 may include materials such as, but not limited to, tungsten or molybdenum.

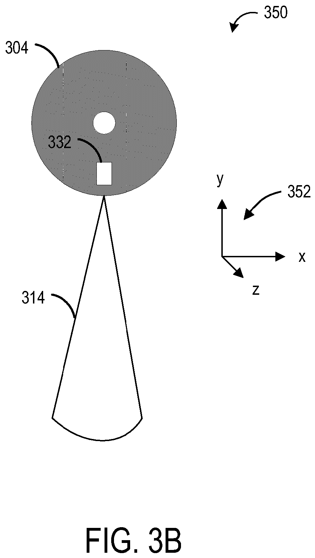

Referring briefly to FIG. 3B, a front view 350 of the target 304 is shown, including the focal spot 332. Focal spot 332 may have an orientation in FIG. 3B indicated by coordinate axes 352. As described above, the target 304 may be a circular target that rotates such that the focal spot 332 is generated at different locations on a surface of the target 304 as the target 304 rotates. By generating the focal spot 332 at different locations on the surface of the target 304, an amount of heat absorbed at a location of the target 304 may be minimized.

Returning to FIG. 3A, size of a focal spot on the target 304 may be adjusted to reduce an amount of heat generated in the target 304, where a smaller focal spot may generate a greater amount of heat at a specific location. An electron collector 329, held at a same potential as the target 304, serves as a sink of electrons that bounce off the surface of 304 during the initial impact, which reduces the chance of those same electrons re-striking the target. Collecting the backscattered electrons in this way further may reduce target heating.

The X-ray tube 300 may include one or more focusing electrodes 316, which may be disposed adjacent to the emitter 308 such that the one or more focusing electrodes 316 focus the electron beam 312 towards the target 304. As used herein, the term "adjacent" means near to in space or position. To focus the electron beam 312, voltages may be applied to the one or more focusing electrodes 316 to generate the one or more electric fields 321. The voltages may be different for each of the one or more focusing electrodes 316. In some embodiments, a first portion of the focusing electrodes 316 may be used for deflecting the electron beam 312, and a second portion of the focusing electrodes 316 may be used for focusing the electron beam 312. In this way, the voltages may be selectively applied by a controller of a control electronics module 322 to generate one or more specific electric fields that focus the electron beam 312 to a desired shape and deflect the electron beam 312 to a desired position. Additionally, the X-ray tube 300 may include one or more extraction electrodes 318, which may be used for additionally controlling and focusing the electron beam 312 towards the anode 303.

As the electron beam 312 is focused on the target 304, the electrons may form a Gaussian distribution. For the purposes of this disclosure, the Gaussian distribution may be an approximately Gaussian distribution. The Gaussian distribution of electrons of the electron beam 312 may be narrowed or parallelized, where electrons colliding with the target 304 at sides of the Gaussian distribution may be directed towards a center of the Gaussian distribution. Said a different way, a distribution of electrons at the sides of the Gaussian distribution may be inverted, resulting in a focal spot of a rectangular shape or batwing shape.

Additionally, the X-ray tube 300 may also include a one or more magnets 324 for focusing and/or positioning and deflecting the electron beam 312 onto the target 304. In various embodiments, the one or more magnets 324 may be disposed between the cathode 302 and the target 304.

As properties of the electron beam current and voltage change, electrostatic focusing of the electron beam 312 will change accordingly. In order to maintain a stable size, shape, and other characteristics of a focal spot, or quickly modify focal spot size and/or shape according to system requirements, the one or more magnets 324 may provide a magnetic field having a performance controllable from steady-state to a sub-30 microsecond time scale for a wide range of focal spot sizes and shapes. When the electron beam 312 has been focused and positioned, the electron beam 312 impinges upon the target 304 at a focal spot 332 to generate the X-rays 314. The X-rays 314 generated by collision of the electron beam 312 with the target 304 may be directed from the X-ray tube 300 through an opening in the tube casing 306, at an X-ray window 337, towards an object 328.

As a result of the electron beam 312 colliding with target 304 at the focal spot 332, a set of X-rays 336 may be generated and directed out X-ray window 337 towards the object 328. The set of X-rays 336 may intersect with the object 328 at an effective focal spot 340. The effective focal spot may have a width (in an X dimension, as indicated by coordinate axes 348) and a length (in an Z dimension, as indicated by the coordinate axes 348).

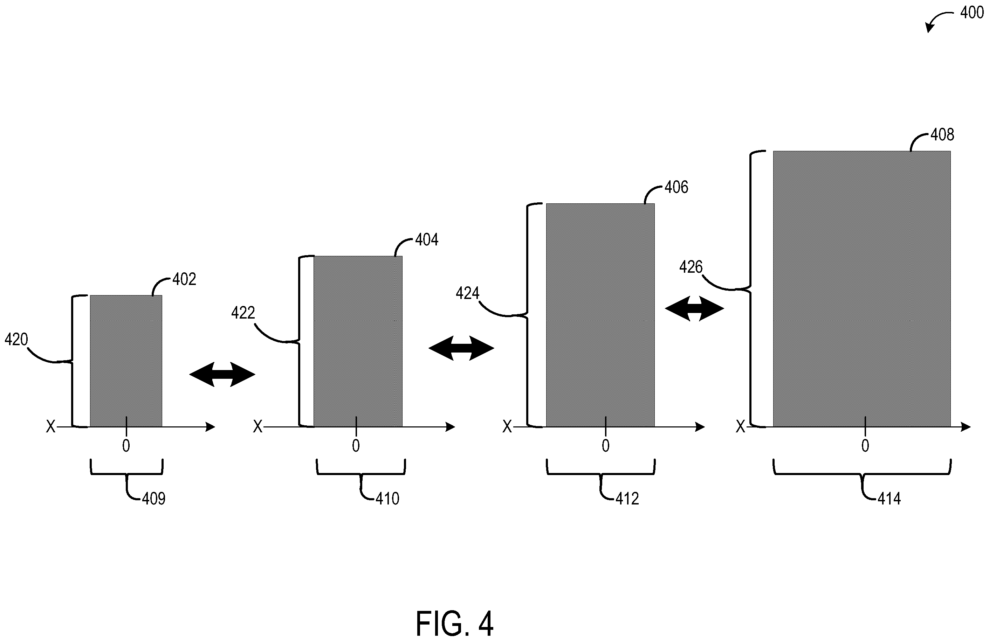

As described above, X-ray tubes may be designed to produce a number of discrete focal spots that can be individually selected for a scan. For example, the CT system may support four different focal spot sizes: an extra small focal spot, a small focal spot, a large focal spot, and an extra-large focal spot, as shown in FIG. 4.

Referring now to FIG. 4, a diagram 400 shows a distribution of electrons of an electron beam (e.g., electron beam 312) of a PCCT system focused on a first focal spot 402 of a first size, a second focal spot 404 of a second size, a third focal spot 406 of a third size, and a fourth focal spot 408 of a fourth size, which may each be the same as or similar to the focal spot 332 of FIG. 3A on the target 304. In various embodiments, the focal spots 402, 404, 406, and 407 may be examples of discrete focal spot sizes supported by the PCCT system, where other focal spot sizes may not be supported by the PCCT system.

A size and shape of the focal spots 402, 404, 406, and 407 may depend on one or more electric fields or electromagnetic fields (e.g., the one or more electric fields 321) generated by one or more focusing electrodes (e.g., the one or more focusing electrodes 316). For example, the one or more electric fields may perform a first focusing of an electron beam (e.g., electron beam 312) to generate the first focal spot 402. The one or more electric fields may perform a second focusing of the electron beam to generate the second focal spot 404. The one or more electric fields may perform a third focusing of the electron beam to generate the third focal spot 406. The one or more electric fields may perform a fourth focusing of the electron beam to generate the fourth focal spot 408. In some embodiments, size and shape of the focal spots 402, 404, 406, and 407 may also depend on one or more magnetic fields (e.g., the magnetic fields 323) generated by one or more magnets (e.g., the one or more magnets 324), which may further focus the electron beam after focusing is performed by the one or more electric fields.

The distributions of electrons generating the focal spots 402, 404, 406, and 407 are depicted as having a shape that is rectangular. While the rectangular shape may be approximated on the target as a result of a configuration of the one or more electric fields, the one or more magnetic fields, and various shields or barriers of the PCCT system, it should be appreciated that the distribution of electrons throughout the focal spots may not be uniform, but rather based on combinations of distributions generated by the configuration of the one or more electric fields and/or the one or more magnetic fields.

The first focal spot 402 has a length 420 and a width 409, as measured along an X-axis of the first focal spot 402. Thus, the first focal spot 402 has a first size based on the length 420 and the width 409. The first focal spot 402 may be referred to as an extra-small (XS) focal spot. The second focal spot 404 may have a different length 422, and have a different width 410, where width 410 is greater than width 409 and length 422 is greater than length 420. The second focal spot 404 may be referred to as a small (S) focal spot. Similarly, the third focal spot 406 may have a width 412 and a length 424, which may be greater than width 410 and length 420, respectively, which may be referred to as a large (L) focal spot. The fourth focal spot 408 may have a width 414 and a length 426, which may be greater than width 412 and lengths 424, respectively.

The size of the focal spots 402, 404, 406, and 408 may be adjusted in various ways. As an emitter is activated with a predetermined amount of current, a first amount of energy (e.g., current) delivered to the one or more focusing electrodes and a second amount of energy delivered to the one or more magnets may each be independently controlled to focus the electron beam on the target to achieve a desired size.

A quality of an image reconstructed by the PCCT system may depend on a selected focal spot size for a corresponding acquisition. In other words, when other scanning parameters are held constant, using the first focal spot 402 may result in a first image of a first quality; using the second focal spot 404 may result in a second image of a second quality; using the third focal spot 406 may result in a third image of a third quality; and using the fourth focal spot 408 may result in a fourth image of a fourth quality, where the first, second, third, and fourth qualities may be different. A scanning protocol may dictate what size focal spot to use for a given acquisition. A typical range of focal spot size is from 0.3mm to 2mm. The smaller the focal spot size is, the better spatial resolution is expected. However, a smaller focal spot size limits scan mA range since it can heat up the small X-ray target area and can damage it. After projection data is acquired using the selected focal spot size/shape, one or more calibration vectors indicated by the scanning protocol may be applied to the projection data to correct for variance in detector responses.

The one or more calibration vectors may include air calibration vectors and MD calibration vectors, which may depend on the selected focal spot size. Typically, a first MD calibration vector is applied for a combination of scanning parameters when the first focal spot 402 is used; a second, different MD calibration vector is applied for the combination when the second focal spot 404 is used; a third, different MD calibration vector is applied for the combination when the third focal spot 406 is used; and a fourth, different MD calibration vector is applied for the combination when the fourth focal spot 408 is used, where the first, second, third, and fourth MD calibration vectors are generated and stored in a memory of the PCCT system during a calibration of the PCCT system.

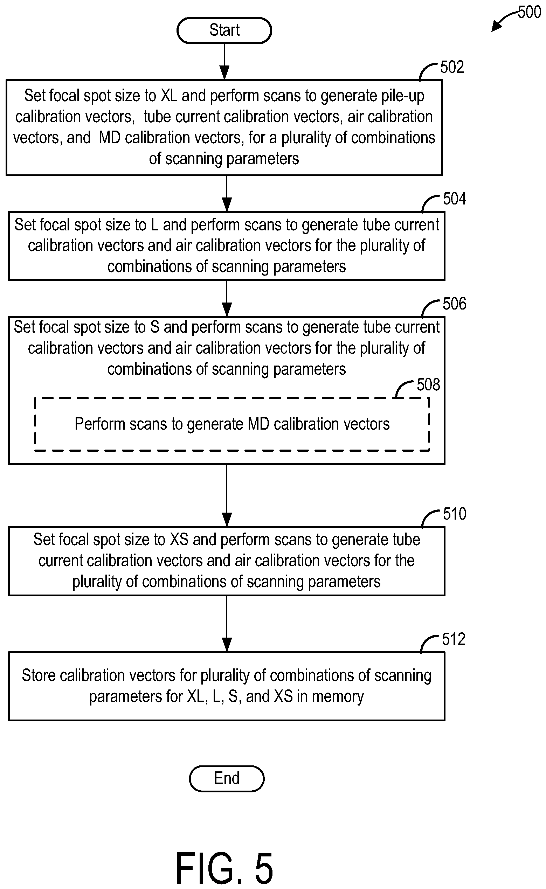

However, because a different MD calibration vector may be selected for each combination of a plurality of combinations of other scanning parameters, an amount of MD calibration vectors stored in the memory may be large, and an amount of time taken to generate the amount of MD calibration vectors during a calibration stage may be long. To reduce the amount of different MD calibration vectors stored in the memory, and to reduce the amount of time taken to generate the MD calibration vectors, an alternative procedure for correcting the projection data using calibration vectors is proposed based on the methods shown in FIGS. 5-7.

Referring now to FIG. 5, a method 500 is shown for acquiring a plurality of calibration vectors for correcting projection data acquired using a PCCT system, such as the PCCT systems 100 and 200 of FIGS. 1 and 2, respectively. The calibration vectors may be acquired during a periodic or regular calibration process performed on the PCCT system. Method 500 and other methods described herein may be performed by a processor of a computing device of the PCCT system, such as computing device 216 of FIG. 2.

Method 500 begins at 502, where method 500 includes setting a scanning parameter for a focal spot size to extra-large (XL), and performing a plurality of acquisitions to generate a corresponding plurality of calibration vectors. The plurality of calibration vectors may include pile-up calibration vectors, tube current calibration vectors, air calibration vectors, and MD calibration vectors, as well as other types of calibration vectors. The plurality of acquisitions may be performed for a plurality of combinations of scanning parameters, including aperture size, tube voltage, bowties, and focal spot sizes. For example, a first acquisition may be performed with the XL focal spot, a first aperture size, a first tube voltage, and a first bowtie parameter; a second acquisition may then be performed with the XL focal spot, a second aperture size, a second tube voltage, and a second bowtie parameter; a third acquisition may then be performed with the XL focal spot, a third aperture size, a third tube voltage, and a third bowtie parameter; and so on.

In various embodiments, the parameters for aperture size, tube voltage, and bowties may be adjusted by increments for each acquisition, over predetermined increment ranges for each parameter type. In this way, a set of different combinations of parameter settings may be used for each acquisition until the parameter ranges have been covered, to acquire a set of calibration vectors corresponding to the different combinations of parameter settings. For example, four different aperture sizes may be considered (e.g., 5 mm, 20 mm, 40 mm, 80 mm); three different tube voltages may be considered (e.g., 80 kVps, 120 kVps, 140 kVps); two different bowtie parameters may be considered (e.g., small, large); and four different focal spots may be considered (XS, S, L, XL). Because focal spot size may account for the most variance in detector response, the focal spot size may be set first (e.g., initially at XL), and calibration vectors for each combination of the other scanning parameters may be generated before changing to a different focal spot size.

At 504, method 500 includes setting the scanning parameter for focal spot size to large (L), and performing the plurality of acquisitions described at step 502, to generate a corresponding plurality of calibration vectors for the different combinations of parameter settings for the L focal spot. However, for the L focal spot, acquisitions may be performed to acquire the air calibration vectors and the tube current calibration vectors, but acquisitions may not be performed for the pile-up calibration vectors and the MD calibration vectors. (Pile-up calibration is independent of focal spot size.) By not performing the acquisitions for the pile-up and MD calibration vectors, an amount of calibration data collected by the PCCT system may be reduced. As a result, a first amount of time taken to perform step 504 may be significantly less than a second amount of time taken to perform step 502, and an overall calibration time may be reduced. In such cases, the XL focal spot MD may be used for the image reconstruction of L or other smaller focal spot scan data.

At 506, method 500 includes setting the scanning parameter for focal spot size to small (S), and performing the plurality of acquisitions described at step 504, to generate a corresponding plurality of calibration vectors for the different combinations of parameter settings for the S focal spot. In some embodiments, for the S focal spot, acquisitions may be performed to acquire the air calibration vectors and the tube current calibration vectors, but acquisitions may not be performed for the MD calibration vectors, to reduce the amount of time taken to perform step 506 and to reduce the amount of MD calibration data collected during the acquisitions. However, in other embodiments, at 508, scans may be performed to generate the MD calibration vectors as described above in reference to step 502, which may increase a quality of images reconstructed from the projection data when smaller focal spots, like XS, are used.

At 510, method 500 includes setting the scanning parameter for focal spot size to extra-small (XS) and performing the plurality of acquisitions described at step 504, to generate a corresponding plurality of calibration vectors for the different combinations of parameter settings for the S focal spot. As in step 504, acquisitions may be performed to acquire the air calibration vectors and the tube current calibration vectors, but acquisitions may not be performed for the MD calibration vectors, to reduce the amount of time taken to perform step 506 and to reduce the amount of MD calibration data collected during the acquisitions.

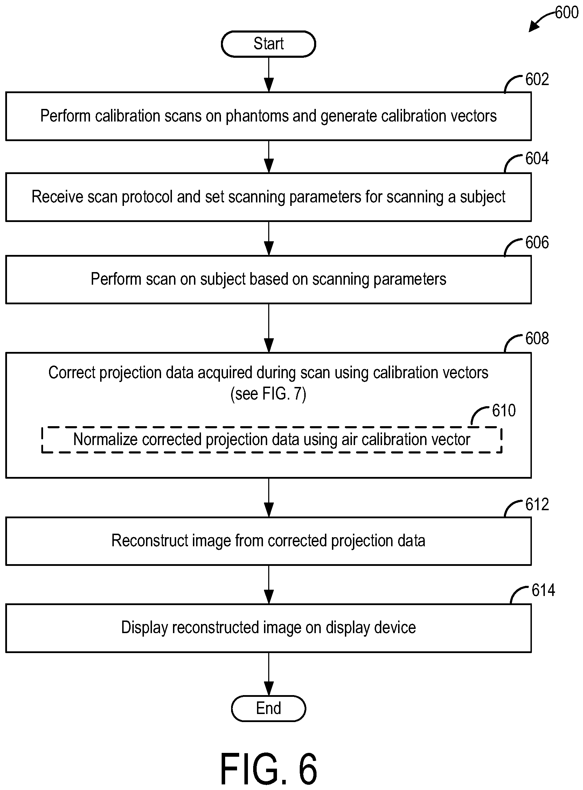

At 512, method 500 includes storing the calibration vectors for the plurality of combinations of scanning parameters for each of the XL, L, S, and XS focal spot sizes in a memory of the PCCT system. The calibration vectors may be retrieved during a scan of the subject using the PCCT system, and applied to projection data acquired during the scan as described below in reference to method 600 of FIG 6. Method 500 ends.

Referring now to FIG. 6, a method 600 is shown for correcting projection data of a subject acquired using a PCCT system, such as the PCCT systems 100 and 200 of FIGS. 1 and 2, respectively, by applying one or more calibration vectors stored in a memory of the PCCT system to the projection data.

Method 600 begins at 602, where method 600 includes performing a plurality of calibration scans on one or more phantoms, to generate the one or more calibration vectors. The calibration vectors may be generated and stored in a memory of the PCCT system prior to performing a scan on the subject, for example, during a previous periodic or regular calibration process performed on the PCCT system, as described above in reference to method 500 of FIG. 5. For example, one or more calibration vectors may be acquired and stored during a calibration process performed on the PCCT system on a morning of a day of the scan, as part of a daily calibration.

Sets of calibration scans may be performed using various combinations of parameter settings at each of four focal spot sizes. In particular, a first set of calibration scans may be performed using a first focal spot of a first size, including calibration scans for generating pile-up calibration vectors, MD calibration vectors, air calibration vectors, and tube current calibration vectors to correct for variance in detector output at the various combinations of parameter settings. Additional sets of calibration scans may be performed using different focal spot sizes, including calibration scans for generating the air calibration vectors and tube current calibration vectors for the various combinations of parameter settings, but not including calibration scans for generating the MD calibration vectors. By not performing the calibration scans for generating the MD calibration vectors at each of the different focal spot sizes, an amount of calibration data stored in the memory of the PCCT system may be reduced, thereby increasing an availability of the memory for other data and tasks. Additionally, a time spent performing the calibration scans for generating the MD calibration vectors at each of the different focal spot sizes may be saved, shortening the calibration stage and increasing an availability of the PCCT system for use on subjects.

At 604, method 600 includes receiving a scan protocol and setting a plurality of scanning parameters of the PCCT system to perform a scan on a subject, based on the scan protocol. In various embodiments, the scan protocol may be selected by an operator of the PCCT system.

At 606, method 600 includes performing a scan of the subject based on the plurality of scanning parameters. The plurality of scanning parameters may include a size of a focal spot of the PCCT system. Additionally, the plurality of scanning parameters may include one or more of an aperture size, a tube current, a collimation parameter, and/or other parameters of the PCCT system. Projection data of the subject is acquired as the scan is performed.

At 608, method 600 includes correcting for variance in detector output during the scan by applying a set of calibration vectors to the acquired projection data. The set of calibration vectors may include a pile-up calibration vector, a tube current calibration, an air calibration vector, and an MD calibration vector. The set of calibration vectors may be retrieved from the memory of the PCCT system, using one or more lookup tables. The one or more lookup tables may indicate the set of calibration vectors to apply to the acquired projection data based on the plurality of scanning parameters used for performing the scan.

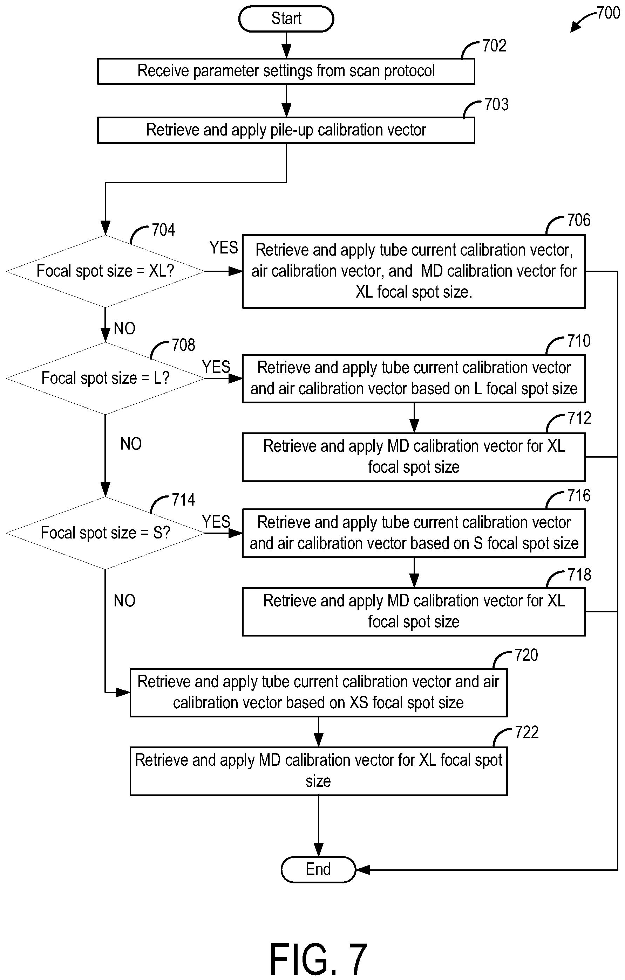

First, a pile-up correction that is independent of focal spot size is applied to the acquired projection data. Second, the tube current and air calibration vectors corresponding to the first focal spot size of the projection data is applied. Using the matching air calibration vector, the acquired projection data is compensated for the focal size difference in the MD vector. Basically, the input projection data to the MD calibration is corrected for the focal size difference in the detector response or MD calibration reduction. Third, an MD calibration vector is applied to the acquired projection data, based on a first size of a focal spot used for the scan, where the indicated MD calibration vector may have been generated for a focal spot of a second size different from the first size. This procedure for retrieving and applying the one or more calibration vectors is described in greater detail in reference to FIG. 7.

Because of the dependence of the MD correction on focal spot size, using the MD calibration vector generated for the different sized focal spot may reduce an effectiveness of the MD correction. Thus, at 610, method 600 optionally includes correcting and normalizing the projection data using the air calibration vector to compensate for the effect generated by the MD reduction. This correction and normalization may occur before the MD correction. During the normalization using the air calibration vector, an air calibration correction value is multiplied by a photon count at each energy bin of the corresponding detector, for each pixel.

In some embodiments, the air calibration vector normalization may be performed based on a sum of the air calibration correction values from each energy bin of each detector. That is, for each energy bin $i$ of a total of $n$ energy bins of a given pixel, the projection data for the energy bin may be divided by the sum of the air calibration correction values from each energy bin of the detector, as described by the following expression 1:

$$\frac{scandata(pixel, energy\ bins\_i)}{\sum_i^n aircal(pixel, energy\ bins\_i)} \qquad (1)$$

In other embodiments, the projection data may be normalized by the corresponding air calibration vector on a bin-by-bin basis. That is, for each energy bin $i$ of a total of $n$ energy bins of a given pixel, the projection data for the energy bin may be divided by the air calibration correction value for the corresponding energy bin, as described by the following expression 2:

$$\frac{scandata(pixel, energy\ bins\_i)}{aircal(pixel, energy\ bins\_i)} \qquad (2)$$

For example, when an MD calibration vector for an XL focal spot is used to correct projection data acquired with a L focal spot, the resulting spectral difference may be compensated by a bin-by-bin air calibration vector normalization as described by the following expression 3:

$$\frac{L\_scandata(pixel, energy\ bins\_i)}{\sum_i^n L\_air(pixel, energy\ bins\_i)} * \frac{\frac{XL\_air(pixel, energy\ bins\_i)}{\sum_i^n XL\_air(pixel, energy\ bins\_i)}}{\frac{L\_air(pixel, energy\ bins\_i)}{\sum_i^n L\_air(pixel, energy\ bins\_i)}} \qquad (3)$$

Thus, for each energy bin of a relevant pixel/detector, a ratio is calculated between a relevant air calibration correction value of the XL air calibration vector for the energy bin normalized by a sum of the XL air calibration correction values of each energy bin of the detector, and a relevant air calibration correction value of the L air calibration vector for the energy bin normalized by a sum of the L air calibration correction values of each energy bin of the detector. The projection data is then divided by the sum of the air calibration correction values of the L air calibration vector for each energy bin of a relevant detector, and then multiplied by the ratio to obtain a normalized photon count at the energy bin of the relevant detector. In this way, the projection data is compensated for a change in photon counts caused by the focal spot size difference in MD. The MD calibration vector is then applied to the compensated projection data to generate one or more material decomposed sinograms.

At 612, method 600 includes reconstructing an image from the corrected projection data (e.g., the material decomposed sinograms), and at 614, displaying the reconstructed image on a display device. For example, the display device may be display device 232 of the imaging system 200 of FIG. 2.

Referring now to FIG. 7, a method 700 is shown for determining a set of vectors to be applied to projection data acquired from a subject using a PCCT system, such as the PCCT systems 100 and 200 of FIGS. 1 and 2, respectively. In various embodiments, method 700 may be performed as part of method 600 of FIG. 6 described above.

At 702, method 700 includes receiving parameter settings of a scan performed on the subject, from a scan protocol used for the scan. The received parameter settings may include, for example, a focal spot size parameter, an aperture parameter, a bowtie parameter, a tube voltage parameter, a tube current parameter, a collimation parameter, and/or other parameters.

At 703, method 700 includes retrieving and applying a pile-up calibration vector. The pile-up calibration vector is not dependent on focal spot size.

At 704, method 700 includes determining whether a size of a focal spot used for the scan is XL. If it is determined that the size the focal spot is XL, method 700 proceeds to 706. At 706, method 700 includes retrieving and applying at least a tube current calibration vector, and air calibration vector, and an MD calibration vector associated with the scanning parameters used in the scan, for the XL focal spot. Alternatively, if it is determined that the size of the focal size is not XL, method 700 proceeds to 708.

At 708, method 700 includes determining whether a size of a focal spot used for the scan is L. If it is determined that the size the focal spot is L, method 700 proceeds to 710.

At 710, method 700 includes retrieving and applying a tube current calibration vector and an air calibration vector associated with the combination of scanning parameters used in the scan, for the L focal spot. At 712, method 700 includes retrieving and applying an MD calibration vector associated with the scanning parameters used in the scan, but for the XL focal spot. In other words, an MD calibration vector associated with the combination of scanning parameters used in the scan for the L focal spot are not retrieved and applied, and the MD calibration vector associated with the scanning parameters used in the scan for the XL focal spot are substituted for the MD calibration vector associated with the combination of scanning parameters used in the scan for the L focal spot. Using the MD calibration vector associated with the scanning parameters for the XL focal spot rather than the L focal spot actually used in the scan may reduce an image quality of an image reconstructed from the corrected projection data; however, the projection data corrected using the MD calibration vector associated with the scanning parameters for the XL focal spot may be advantageously normalized using an air calibration vector for the L focal spot to offset the reduction in image quality, as described above in reference to method 600.

Alternatively, if at 708 it is determined that the size of the focal size is not L, method 700 proceeds to 714. At 714, method 700 includes determining whether a size of a focal spot used for the scan is S. If it is determined that the size the focal spot is S, method 700 proceeds to 716.

At 716, method 700 includes retrieving and applying a tube current calibration vector and an air calibration vector associated with the scanning parameters used in the scan, for the S focal spot. At 718, method 700 includes retrieving and applying an MD calibration vector associated with the scanning parameters used in the scan, but for the XL focal spot. In other words, an MD calibration vector associated with the combination of scanning parameters used in the scan for the S focal spot are not retrieved and applied, and the MD calibration vector associated with the combination of scanning parameters used in the scan for the XL focal spot are substituted for the MD calibration vector associated with the combination of scanning parameters used in the scan for the S focal spot.

If at 714 it is determined that the focal spot size is not S, it may be inferred that the focal spot size is XS, whereby method 700 proceeds to 720. At 720, method 700 includes retrieving and applying a tube current calibration vector and an air calibration vector associated with the scanning parameters used in the scan, for the XS focal spot. At 722, method 700 includes retrieving and applying an MD calibration vector associated with the combination of scanning parameters used in the scan, but for the XL focal spot, as in steps 712 and 718. In other words, an MD calibration vector associated with the combination of scanning parameters used in the scan for the XS focal spot are not retrieved and applied, and the MD calibration vector associated with the combination of scanning parameters used in the scan for the XL focal spot are substituted for the MD calibration vector associated with the combination of scanning parameters used in the scan for the XS focal spot. Method 700 ends.

It should be appreciated that while method 700 is described as using an MD calibration vector associated with an XL focal spot for correcting projection data acquired at the L, S, and XS focal spot sizes, an MD calibration vector associated with any of the XL, L, S, and XS focal spots may be advantageously used to correct projection data at other focal spot sizes by following method 700. For example, an MD calibration vector associated with an L focal spot may be used to correct projection data acquired at an XL focal spot size, an S focal spot size, and/or an XS focal spot size; an MD calibration vector associated with an S focal spot may be used to correct projection data acquired at an XS focal spot size, an L focal spot size, and/or an XL focal spot size; and so on.

Additionally or alternatively, method 700 may include retrieving and applying MD calibration vectors associated with more than one focal spot to correct projection data acquired at other focal spots sizes. For example, in one embodiment, projection data acquired using an L focal spot may be corrected by a pile-up calibration vector and an MD calibration vector associated with an XL focal spot (e.g., steps 710-712), and projection data acquired using an XS focal may be corrected by a pile-up calibration vector and an MD calibration vector associated with an S focal spot (e.g., modifying steps 720-722). Such cases may rely on MD calibration vectors being collected for scanning parameter combinations at both of the XL and the S focal spot sizes during the calibration stage, which may increase a quality of images reconstructed at the smaller focal spot sizes, at the cost of longer calibration times and greater amounts of calibration data being stored.

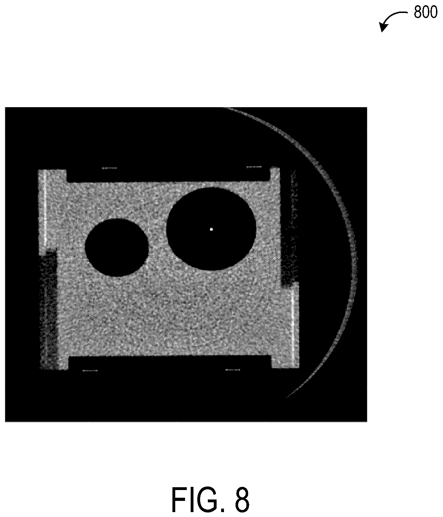

FIGS. 8, 9, and 10 show exemplary images reconstructed from projection data that has been corrected as described herein. Referring to FIG. 8, a first reconstructed image 800 of a phantom is shown, where image 800 is reconstructed from projection data acquired with an XS focal spot, and corrected using an XS pile-up calibration vector, an XS tube current calibration vector, an XS air calibration vector, and an XS MD calibration vector. In other words, the calibration vectors applied to correct the projection data for variance in detector response are generated, during a prior calibration stage, for the XS focal spot size. As a result, an image quality of image 800 is high (e.g., at a desired level).

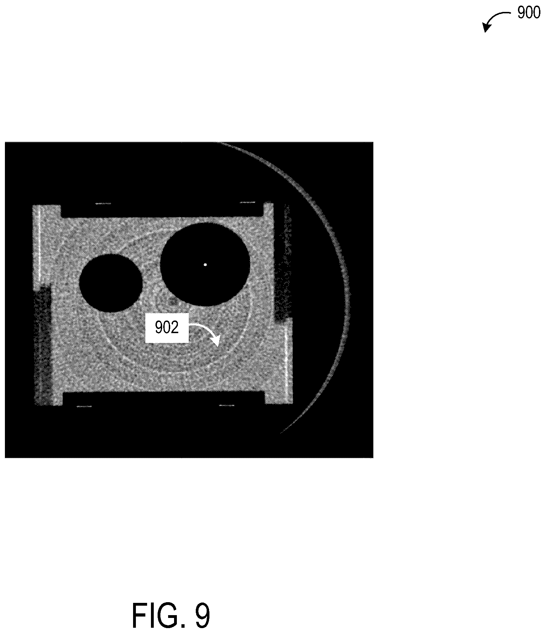

In contrast to FIG. 8, FIG. 9 shows a second reconstructed image 900 of the phantom, which is reconstructed from projection data acquired with the same scanning parameters as image 800, including the XS focal spot size. However, in image 900, the projection data is corrected using an XL pile-up calibration vector, an XL tube current calibration vector, an XL air calibration vector, and an XL MD calibration vector. As a result of using the XL calibration vectors for the XS focal spot size, image 900 is of a lower quality than image 800, where ring artifacts 902 are more visible than in image 800. Thus, image 900 represents a scenario where calibration vectors generated for a different focal spot size are substituted for calibration vectors generated for a focal spot size used in a scan, but without performing the air calibration procedure described in FIG. 6 to adjust the X-ray count to normalize for the focal size difference (e.g., the XL air calibration vector does not correspond to the focal spot used in the scan).

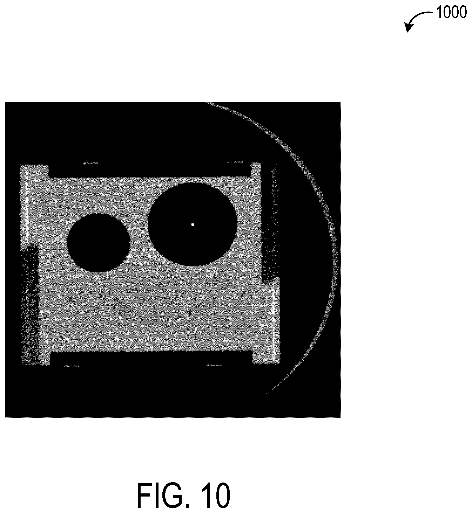

In contrast to FIG. 9, FIG. 10 shows a third reconstructed image 1000 of the phantom, which is reconstructed from projection data acquired with the same scanning parameters as image 800, including the XS focal spot size. However, in image 1000, the projection data is corrected using an XL pile-up calibration vector and an XL MD calibration vector, but an XS tube current calibration vector and an XS air calibration vector. The XS air calibration vector, which corresponds to the XS focal spot used in the scan, is used to normalize the projection data as described above in relation to the air calibration normalization of FIG. 6. As a result of using the air calibration vector for the XS focal spot size to normalize the projection data, the quality of image 1000 is increased with respect to image 900, and similar to image 800. However, because the correction of the projection data in image 1000 does not rely on stored MD, less memory of the PCCT system is taken up, and a time spent generating the calibration vectors is reduced. Thus, by using the approach taken in FIG. 10, an efficiency of the PCCT system is increased without a corresponding sacrifice in image quality.

Thus, as described herein, an overall amount of calibration data (e.g., calibration vectors) stored in a memory of the PCCT system, and a corresponding time spent calibrating the PCCT system may be advantageously reduced, by reducing a total number of MD calibration vectors relied on by the PCCT system for compensating for variance in material densities of portions of an anatomy of a subject scanned by the PCCT system. Rather than generating different sets of calibration vectors for each focal spot size, a single set of MD calibration vectors may be generated that may be applied to various focal spot sizes to correct the projection data. To compensate for spectral differences in photon counts resulting from using an MD calibration vector of the single set of MD calibration vectors (e.g., of a single focal spot size) to correct projection data acquired with a different focal spot size, rather than using an MD calibration vector generated based on different focal spot size, the corrected projection data may be normalized by multiplying a ratio of air calibration corrections corresponding to the actual focal spot size used during the scan to air calibration corrections corresponding to the substituted focal spot size.

By using the single set of MD calibration vectors to correct the projection data acquired at each focal spot size and normalizing the corrected projection data using the air calibration vectors, the time spent performing the calibrations and an amount of MD calibration data collected may be significantly decreased, without a corresponding reduction in quality of a reconstructed image. As a result of the reduction in the time spent performing the calibrations, the PCCT system may have greater availability for patient scans. Further, by reducing the amount of computational and memory resources consumed by the PCCT system during a calibration stage of the PCCT system, an efficiency of the functioning of PCCT system is increased, where a same or similar result of a scan may be obtained with less computation. The saved computational and memory resources may then be advantageously used for other imaging tasks and/or on other subjects. The technical effect of correcting projection data acquired via the PCCT system using an MD vector of a single focal spot size, rather than using an MD vector generated for a specific size focal spot, is that an amount of calibration data stored in the PCCT system and a corresponding amount of time spent generating the calibration vectors may be reduced, increasing an efficiency of the use of the PCCT system.

The disclosure also provides support for a method for a photon counting computed tomography (PCCT) system, the method comprising: performing a scan using the PCCT system, with a first focal spot of a first size, applying a material decomposition (MD) calibration vector to correct projection data acquired during the scan, the material decomposition (MD) calibration vector generated for a second focal spot of a second size, the second size different from the first size, reconstructing an image from the corrected projection data, and displaying the image on a display device. In a first example of the method, the method further comprises: correcting and normalizing the projection data using a first air calibration vector generated for the first focal spot of the first size and a second air calibration vector generated for the second focal spot of the second size. In a second example of the method, optionally including the first example, correcting and normalizing the projection data using the first and second air calibration vectors further comprises, for each energy bin of a total number of energy bins of each detector of the PCCT system, dividing the projection data for the energy bin by the sum of the air calibration correction values of the first air calibration vector for each energy bin of the detector. In a third example of the method, optionally including one or both of the first and second examples, correcting and normalizing the projection data using the first and second air calibration vectors further comprises, for each energy bin of a total number of energy bins of each detector of the PCCT system, dividing the projection data for the energy bin by the air calibration correction value of the first air calibration vector for the energy bin. In a fourth example of the method, optionally including one or more or each of the first through third examples, correcting and normalizing the projection data using the air calibration vector further comprises: for each energy bin of each detector of the PCCT system: calculating a ratio between a first air calibration correction value of the second air calibration vector for the energy bin normalized by a sum of the air calibration correction values of the second air calibration vector for each energy bin of the detector, and a second air calibration correction value of the first air calibration vector for the energy bin normalized by a sum of the air calibration correction values of the first air calibration vector for each energy bin of the detector, and dividing the corrected projection data for the energy bin by the sum of the air calibration correction values of the first air calibration vector for each energy bin of a relevant detector, and then multiplying the result by the ratio to obtain a normalized photon count at the energy bin of the detector. In a fifth example of the method, optionally including one or more or each of the first through fourth examples, the method further comprises: applying a pile-up calibration vector to correct projection data acquired during the scan, the pile-up calibration vector generated not dependent on focal spot size. In a sixth example of the method, optionally including one or more or each of the first through fifth examples, the first focal spot of the first size is a large (L) focal spot, and the second focal spot of the second size is an extra-large (XL) focal spot. In a seventh example of the method, optionally including one or more or each of the first through sixth examples, the first focal spot of the first size is an extra-small (XS) focal spot, and the second focal spot of the second size is a small (S) focal spot. In an eighth example of the method, optionally including one or more or each of the first through seventh examples,: in a first condition where the first focal spot is an XL focal spot, a first MD calibration vector associated with the XL focal spot is applied to the projection data, and in a second condition where the first focal spot is an L focal spot, a second MD calibration vector associated with the L focal spot is not applied, and the first MD calibration vector associated with the XL focal spot is applied to the projection data. In a ninth example of the method, optionally including one or more or each of the first through eighth examples,: in a first condition where the first focal spot is an S focal spot, a third MD calibration vector associated with the S focal spot is applied to the projection data, and in a second condition where the first focal spot is an XS focal spot, a fourth MD calibration vector associated with the XS focal spot is not applied, and the third MD calibration vector associated with the S focal spot is applied to the projection data.

The disclosure also provides support for a photon counting computed tomography (PCCT) system, comprising: an X-ray source that emits a beam of X-rays toward a subject to be imaged, a photon counting detector that receives the beam of X-rays attenuated by the subject, a data acquisition system (DAS) operably connected to the detector, and a computer comprising a processor and a non-transitory memory operably connected to the DAS, wherein instructions are stored in the non-transitory memory that when executed cause the processor to: during a scan performed using the PCCT system: collect a photon count at each detector of the PCCT system, apply a pile-up calibration correction value, a tube current calibration correction value, an air calibration correction value, and a material decomposition (MD) calibration correction value to the photon count to obtain one or more material decomposed sinograms, reconstruct an image based on the one or more material decomposed sinograms, and output the image to a display device, wherein the air calibration correction value and the tube current calibration correction value are based on a first focal spot size used when scanning the view, and the MD calibration correction value is based on a second focal spot size not used when scanning the view, the second focal spot size different from the first focal spot size. In a first example of the system, the pile-up calibration correction value, the tube current calibration correction value, the air calibration correction value, and the MD calibration correction value are retrieved from a pile-up calibration vector, a first air calibration vector, a tube current calibration vector, and an MD calibration vector, respectively, stored in the non-transitory memory. In a second example of the system, optionally including the first example, at each detector, the pile-up calibration correction value, the tube current calibration correction value, the air calibration correction value, and the MD calibration correction value are applied to generate a material decomposed sinogram. In a third example of the system, optionally including one or both of the first and second examples, the photon count at each energy bin is normalized by a sum of the air calibration correction values across all energy bins of the detector. In a fourth example of the system, optionally including one or more or each of the first through third examples, the photon count at each energy bin is normalized by the air calibration correction value applied at the energy bin. In a fifth example of the system, optionally including one or more or each of the first through fourth examples, further instructions are stored in the non-transitory memory that when executed, cause the processor to: retrieve a second air calibration vector from the non-transitory memory, the second air calibration vector generated for the second focal spot size, and at each energy bin of each detector of the PCCT system: calculate a ratio between a first air calibration correction value of the second air calibration vector for the energy bin normalized by a sum of the air calibration correction values of the second air calibration vector for each energy bin of the detector, and a second air calibration correction value of the first air calibration vector for the energy bin normalized by a sum of the air calibration correction values of the first air calibration vector for each energy bin of the detector, and divide the corrected projection data for the energy bin by the sum of the air calibration correction values of the first air calibration vector for each energy bin of a relevant detector, and multiply the result by the ratio to obtain a normalized photon count at the energy bin of the detector. In a sixth example of the system, optionally including one or more or each of the first through fifth examples, the first focal spot size is an extra-large (XL) focal spot, and the second focal spot size is one of a large (L) focal spot, a small (S) focal spot, and an extra-small (XS) focal spot. In a seventh example of the system, optionally including one or more or each of the first through sixth examples, further instructions are stored in the non-transitory memory that when executed, cause the processor to: during a calibration stage of the PCCT system: generate and store in the non-transitory memory a set of pile-up calibration vectors, a set of tube current calibration vectors, a set of air calibration vectors, and a set of MD calibration vectors for a focal spot of a first size, and generate and store in the non-transitory memory a set of air calibration vectors and a set of tube current calibration vectors for focal spots of a different size than the first size, and not generate a set of MD calibration vectors for the focal spots of the different size. In a eighth example of the system, optionally including one or more or each of the first through seventh examples, further instructions are stored in the non-transitory memory that when executed, cause the processor to: during the calibration stage: generate and store in the non-transitory memory a set of pile-up calibration vectors, a set of tube current calibration vectors, a set of air calibration vectors, and a set of MD calibration vectors for a first focal spot of the first size and a second focal spot of a second size, and generate and store in the non-transitory memory a set of air calibration vectors and a set of tube current calibration vectors for focal spots of a different size than both of the first size and the second size, and not generate a set of MD calibration vectors for focal spots of a different size than both of the first size and the second size.

The disclosure also provides support for a method for calibrating a photon counting computed tomography (PCCT) system, the method comprising: during a calibration stage of the PCCT system: setting a focal spot size scanning parameter of the PCCT system to a first size, generating a set of pile-up calibration vectors, a set of tube current calibration vectors, a set of air calibration vectors, and a set of MD calibration vectors, storing the set of pile-up calibration vectors, the set of air calibration vectors, the set of tube current calibration vectors, and the set of MD calibration vectors in a memory of the PCCT system, setting the focal spot size scanning parameter of the PCCT system to a one or more different sizes, and for each size of the one or more different sizes: generating and storing in the memory a set of air calibration vectors and a set of tube current calibration vectors, and not generating a set of MD calibration vectors.

When introducing elements of various embodiments of the present disclosure, the articles "a," "an," and "the" are intended to mean that there are one or more of the elements. The terms "first," "second," and the like, do not denote any order, quantity, or importance, but rather are used to distinguish one element from another. The terms "comprising," "including," and "having" are intended to be inclusive and mean that there may be additional elements other than the listed elements. As the terms "connected to," "coupled to," etc. are used herein, one object (e.g., a material, element, structure, member, etc.) can be connected to or coupled to another object regardless of whether the one object is directly connected or coupled to the other object or whether there are one or more intervening objects between the one object and the other object. In addition, it should be understood that references to "one embodiment" or "an embodiment" of the present disclosure are not intended to be interpreted as excluding the existence of additional embodiments that also incorporate the recited features.

In addition to any previously indicated modification, numerous other variations and alternative arrangements may be devised by those skilled in the art without departing from the spirit and scope of this description, and appended claims are intended to cover such modifications and arrangements. Thus, while the information has been described above with particularity and detail in connection with what is presently deemed to be the most practical and preferred aspects, it will be apparent to those of ordinary skill in the art that numerous modifications, including, but not limited to, form, function, manner of operation and use may be made without departing from the principles and concepts set forth herein. Also, as used herein, the examples and embodiments, in all respects, are meant to be illustrative only and should not be construed to be limiting in any manner.

**Claims**

1.  A method (600) for a photon counting computed tomography (PCCT) system, the method (600) comprising:

    performing a scan using the PCCT system, with a first focal spot of a first size (606);
    applying a material decomposition (MD) calibration vector to correct projection data acquired during the scan (608), the material decomposition (MD) calibration vector generated for a second focal spot of a second size, the second size different from the first size;
    reconstructing an image from the corrected projection data (612); and
    displaying the image on a display device (614).

2.  The method (600) of claim 1, further comprising correcting and normalizing the projection data using a first air calibration vector (610) generated for the first focal spot of the first size and a second air calibration vector generated for the second focal spot of the second size.

3.  The method (600) of claim 2, wherein correcting and normalizing the projection data using the first and second air calibration vectors further comprises, for each energy bin of a total number of energy bins of each detector of the PCCT system, dividing the projection data for the energy bin by the sum of the air calibration correction values of the first air calibration vector for each energy bin of the detector.

4.  The method (600) of claim 2, wherein correcting and normalizing the projection data using the first and second air calibration vectors further comprises, for each energy bin of a total number of energy bins of each detector of the PCCT system, dividing the projection data for the energy bin by the air calibration correction value of the first air calibration vector for the energy bin.

5.  The method (600) of claim 2, wherein correcting and normalizing the projection data using the air calibration vector further comprises:
    for each energy bin of each detector of the PCCT system:

    calculating a ratio between a first air calibration correction value of the second air calibration vector for the energy bin normalized by a sum of the air calibration correction values of the second air calibration vector for each energy bin of the detector, and a second air calibration correction value of the first air calibration vector for the energy bin normalized by a sum of the air calibration correction values of the first air calibration vector for each energy bin of the detector; and
    dividing the corrected projection data for the energy bin by the sum of the air calibration correction values of the first air calibration vector for each energy bin of a relevant detector, and then multiplying the result by the ratio to obtain a normalized photon count at the energy bin of the detector.

6.  The method (600, 700) of claim 1, further comprising applying a pile-up calibration vector to correct projection data (703) acquired during the scan, the pile-up calibration vector generated not dependent on focal spot size.

7. The method (600, 700) of claim 1, wherein the first focal spot of the first size is a large (L) focal spot, and the second focal spot of the second size is an extra-large (XL) focal spot.

8. The method (600, 700) of claim 1, wherein the first focal spot of the first size is an extra-small (XS) focal spot, and the second focal spot of the second size is a small (S) focal spot.

9. The method (600, 700) of claim 6, wherein:

in a first condition where the first focal spot is an XL focal spot, a first MD calibration vector associated with the XL focal spot is applied to the projection data (706); and
in a second condition where the first focal spot is an L focal spot, a second MD calibration vector associated with the L focal spot is not applied, and the first MD calibration vector associated with the XL focal spot is applied to the projection data (712).

10. The method (600, 700) of claim 6, wherein:

in a first condition where the first focal spot is an S focal spot, a third MD calibration vector associated with the S focal spot is applied to the projection data; and
in a second condition where the first focal spot is an XS focal spot, a fourth MD calibration vector associated with the XS focal spot is not applied, and the third MD calibration vector associated with the S focal spot is applied to the projection data.

11. A photon counting computed tomography (PCCT) system (100, 200), comprising:

an X-ray source (104) that emits a beam of X-rays toward a subject to be imaged;
a photon counting detector (108) that receives the beam of X-rays attenuated by the subject;
a data acquisition system (DAS) operably connected to the detector; and
a computer (216) comprising a processor and a non-transitory memory operably connected to the DAS, wherein instructions are stored in the non-transitory memory that when executed cause the processor to:
during a scan performed using the PCCT system (100, 200):

collect a photon count at each detector of the PCCT system (100, 200);
apply a pile-up calibration correction value, a tube current calibration correction value, an air calibration correction value, and a material decomposition (MD) calibration correction value to the photon count to obtain one or more material decomposed sinograms;
reconstruct an image (1000) based on the one or more material decomposed sinograms, and
output the image (1000) to a display device (232);
wherein the air calibration correction value and the tube current calibration correction value are based on a first focal spot size used during the scan, and the MD calibration correction value is based on a second focal spot size not used during the scan, the second focal spot size different from the first focal spot size.

12. The PCCT system (100, 200) of claim 11, wherein the pile-up calibration correction value, the tube current calibration correction value, the air calibration correction value, and the MD calibration correction value are retrieved from a pile-up calibration vector, a first air calibration vector, a tube current calibration vector, and an MD calibration vector, respectively, stored in the non-transitory memory.

13. The PCCT system (100, 200) of claim 12, wherein at each detector (108), the pile-up calibration correction value, the tube current calibration correction value, the air calibration correction value, and the MD calibration correction value are applied to generate a material decomposed sinogram.

14. The PCCT system (100, 200) of claim 13, wherein the photon count at each energy bin is normalized by a sum of air calibration correction values across all energy bins of the detector (108).

15. The PCCT system (100, 200) of claim 13, wherein the photon count at each energy bin is normalized by the air calibration correction value applied at the energy bin.

FIG. 1

FIG. 2

FIG. 3A

EP 4 644 887 A1

FIG. 3B

FIG. 4

500

Start

502

Set focal spot size to XL and perform scans to generate pile-up calibration vectors, tube current calibration vectors, air calibration vectors, and MD calibration vectors, for a plurality of combinations of scanning parameters

504

Set focal spot size to L and perform scans to generate tube current calibration vectors and air calibration vectors for the plurality of combinations of scanning parameters

506

Set focal spot size to S and perform scans to generate tube current calibration vectors and air calibration vectors for the plurality of combinations of scanning parameters

508

Perform scans to generate MD calibration vectors

510

Set focal spot size to XS and perform scans to generate tube current calibration vectors and air calibration vectors for the plurality of combinations of scanning parameters

512

Store calibration vectors for plurality of combinations of scanning parameters for XL, L, S, and XS in memory

End

# FIG. 5

600

Start

602

Perform calibration scans on phantoms and generate calibration vectors

604

Receive scan protocol and set scanning parameters for scanning a subject

606

Perform scan on subject based on scanning parameters

608

Correct projection data acquired during scan using calibration vectors
(see FIG. 7)

610

Normalize corrected projection data using air calibration vector

612

Reconstruct image from corrected projection data

614

Display reconstructed image on display device

End

FIG. 6

FIG. 7

800

FIG. 8

FIG. 9

1000

FIG. 10

## EUROPEAN SEARCH REPORT

**Application Number**

EP 25 16 7311

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2023/011644 A1 (ZHAO YING [US]) 12 January 2023 (2023-01-12) * paragraphs [0002], [0028], [0043], [0050], [0327] * * paragraph [0056] - paragraph [0057] * * paragraph [0266] - paragraph [0267] * * paragraph [0420] - paragraph [0450] * * paragraph [0662] - paragraph [0676] * * figures * | 1-15 | INV. G01N23/046 A61B6/42 G01T1/29 G06T11/00 |
| A | US 2018/082818 A1 (MEILER MICHAEL [US] ET AL) 22 March 2018 (2018-03-22) * paragraph [0001] - paragraph [0010] * * paragraph [0055] - paragraph [0062] * * figures * | 1-15 | |
| A | US 2019/247005 A1 (IKHLEF ABDELAZIZ [US] ET AL) 15 August 2019 (2019-08-15) * paragraph [0053] - paragraph [0057]; claims; figures * | 1-15 | |

TECHNICAL FIELDS SEARCHED (IPC)

G01N
A61B
G06T
G01V
G01T

The present search report has been drawn up for all claims

| Place of search | Date of completion of the search | Examiner |
|---|---|---|
| The Hague | 25 September 2025 | Savage, John |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 16 7311

This annex lists the patent family members relating to the patent documents cited in the above-mentioned European search report.
The members are as contained in the European Patent Office EDP file on
The European Patent Office is in no way liable for these particulars which are merely given for the purpose of information.

25-09-2025

| Patent document cited in search report | Publication date | Patent family member(s) | Publication date |
|---|---|---|---|
| US 2023011644 A1 | 12-01-2023 | AU 2020392235 A1 | 02-06-2022 |
| | | CA 3158053 A1 | 03-06-2021 |
| | | CN 114867416 A | 05-08-2022 |
| | | EP 4064995 A1 | 05-10-2022 |
| | | IL 292916 A | 01-07-2022 |
| | | JP 2023503638 A | 31-01-2023 |
| | | KR 20220106811 A | 29-07-2022 |
| | | US 2023011644 A1 | 12-01-2023 |
| | | WO 2021108715 A1 | 03-06-2021 |
| US 2018082818 A1 | 22-03-2018 | CN 107845556 A | 27-03-2018 |
| | | EP 3297017 A1 | 21-03-2018 |
| | | JP 6938815 B2 | 22-09-2021 |
| | | JP 2018049813 A | 29-03-2018 |
| | | US 2018082818 A1 | 22-03-2018 |
| US 2019247005 A1 | 15-08-2019 | NONE | |

For more details about this annex : see Official Journal of the European Patent Office, No. 12/82